# DEMANDE DE BREVET EUROPEEN

(11) **EP 0 895 765 A1**
(43) Date de publication de la demande: **10.02.1999**
(21) Numéro de dépôt: 98401940.6
(22) Date de dépôt: 29.07.1998
(51) Int. Cl.: A61F 9/013

(54) **Appareil chirugical pour réaliser une découpe lamellaire de cornée**

(30) Priorité: 05.08.1997 FR 9710021
(71) Demandeur: Hanna, Khalil, 75007 Paris (FR)
(72) Inventeur: Hanna, Khalil, 75007 Paris (FR)
(74) Mandataire: Robert, Jean-Pierre

(57) **Abrégé**

L'invention concerne un appareil chirurgical pour réaliser une découpe lamellaire de la cornée.

Il comporte :
- une base (1) qui comprend une chambre à dépression annulaire conformée pour être appliquée sur la sclère d'un oeil,
- un élément de conformation de la cornée situé au dessus de la base (1) et présentant une surface (10) tournée en regard de celle-ci,
- une lame de coupe (2) déplaçable dans un plan situé entre la surface (10) de l'élément de conformation et la base (1).

Selon l'invention, l'appareil comporte un support général (3) auquel est attelée la base (1) par des moyens (4,27) lui permettant d'être réglée en position par rapport à ce support (3) le long de l'axe de révolution de la chambre annulaire et auquel est attelée la lame (2) de coupe par des moyens (5) lui permettant d'être réglée en position par rapport au support général (3) le long d'une direction parallèle à l'axe de révolution de la chambre annulaire et d'être déplacée perpendiculairement à cet axe.

## Description

La présente invention concerne un appareil chirurgical -ou kératome- destiné à la chirurgie cornéenne et plus spécialement à la découpe lamellaire de la cornée.

Parmi les techniques de chirurgie réfractive (chirurgie cornéenne) visant à la correction d'une amétropie, on connaît une technique dérivée de la technique mise au point par José BARRAQUER et appelée kératomileusis in situ. Elle consiste à découper un disque de la cornée au moyen d'un microkératome. Ce disque soulevé, on procède ensuite au retrait d'un lenticule sur le plan découvert par le soulèvement du disque, lenticule dont l'épaisseur et les dimensions sont en fonction de la correction à apporter. On replace ensuite le disque cornéen sur le plan ainsi modifié ce qui se traduit par une modification de la surface antérieure de la cornée, et donc de ses caractéristiques optiques.

Cette technique connaît aujourd'hui un regain d'intérêt par l'emploi du laser Eximer, qui permet de réaliser des ablations extrêmement précises. En effet on a pu maîtriser les dimensions des lenticules de manière beaucoup plus fine qu'avec les moyens mécaniques en usage jusqu'alors.

Donc dans ce type d'intervention on retire le disque au moyen d'un microkératome, c'est-à-dire d'un outil proche d'un rabot miniaturisé avec une lame vibrante dans la direction de son fil coupant, et on règle la profondeur de coupe au moyen de cales d'épaisseur précalibrées ou de vis micrométriques.

Tous les appareils disponibles sur le marché comportent deux pièces :
- un anneau de base de l'appareil qui est maintenu sur l'oeil autour de la cornée par une chambre annulaire à dépression,
- un chariot porte couteau qui est manoeuvré soit manuellement soit automatiquement à l'intérieur de glissières prévues à la partie supérieure de l'anneau de fixation.

Les possibilités de réglage et d'ajustement de ces dispositifs sont assez peu nombreuses ou demandent des pièces complémentaires. Pour régler l'épaisseur du disque cornéen, on ajuste la distance séparant le fil de la lame de coupe d'un plateau situé en avant du fil de la lame. Le plateau sert à aplanir la cornée devant l'action de la lame. Pour agir sur le diamètre du disque à découper il faut disposer en général d'un jeu d'anneaux de fixation de différentes dimensions.

Par ailleurs certains appareils proposent un kératome avec une lame vibrante qui se déplace seule entre l'anneau et le plateau d'aplanissement de la cornée. D'autres appareils proposent un kératome dans lequel la lame, qui est inclinée par rapport au plan de coupe, se déplace avec le plateau d'aplanissement de la cornée. Dans ce dernier cas, il convient d'introduire et de déplacer le chariot portant la lame de coupe dans des glissières de guidage de l'anneau, ce qui est une opération délicate, malcommode et peu ergonomique.

La présente invention entend proposer un appareil qui permette au chirurgien de pouvoir procéder aisément à tous les réglages qu'il souhaite et pouvoir choisir entre un appareil à plateau d'aplanissement fixe ou un appareil à plateau d'aplanissement mobile avec la lame de coupe.

A cet effet l'invention a pour objet un appareil chirurgical pour réaliser une découpe lamellaire de la cornée comportant :
- une base qui comprend une chambre à dépression annulaire avec un axe de révolution, conformée pour être appliquée autour de la cornée d'un oeil et pourvue de moyens pour sa connexion à une source de vide,
- un élément de conformation de la cornée situé au-dessus de la base et présentant une surface tournée en regard de celle-ci,
- une lame de coupe déplaçable dans un plan situé entre la surface de l'élément de conformation et la base.

Selon la caractéristique principale de l'invention, cet appareil comporte un support général auquel est attelée la base par des moyens lui permettant d'être réglée en position par rapport à ce support le long de l'axe de révolution de la chambre annulaire et auquel est attelée la lame de coupe par des moyens lui permettant d'être réglée en position par rapport au support général, le long d'une direction parallèle à l'axe de révolution de la chambre annulaire et d'être déplacée perpendiculairement à cet axe.

Ainsi le kératome de l'invention présente-t-il un support général qui constitue un bâti de référence par rapport auquel les éléments du kératome sont mobiles et réglables en position. On peut donc procéder au réglage de manière individuelle de tous les paramètres qui concernent la chirurgie. On notera en particulier que le support de lame est guidé par des moyens qui ne sont pas portés par l'anneau de fixation. Ceci est un avantage de l'invention par rapport aux microkératomes connus. En effet, par construction, l'écartement des glissières portées par l'anneau conditionne le diamètre maximum du disque cornéen à découper qui est, dans les dispositifs connus, quelquefois insuffisant pour par exemple corriger l'hypermétropie. Avec l'invention, cette limite n'existe plus et on peut procéder à la découpe de disques de plus grand diamètre si besoin est.

Dans un mode de réalisation, le support général comporte une bague supérieure formant un moyen de préhension de l'appareil, avec un axe perpendiculaire à son plan, un bras s'étendant à partir de la bague sous celle-ci en convergeant vers son axe, par l'extrémité libre duquel la base est maintenue dans des positions réglables telles que son axe de révolution coïncide avec l'axe de la bague supérieure et par un bras qui s'étend à partir de la bague à l'extérieur de celle-ci et perpendiculairement à son axe, sur lequel est monté un coulissement un chariot porte-lame extensible de manière réglable dans une direction parallèle à l'axe de la bague.

Grâce au support général on peut réaliser un appareil dans lequel soit l'élément de conformation de la cornée est constitué par une lentille rapportée de manière fixe mais démontable au bras convergeant du support général, soit cet élément est solidaire du chariot porte-lame, situé sur ce dernier en avant de la lame dans le mouvement qui tend à rapprocher celle-ci de l'axe général du support. L'une de ces dispositions est bien entendu exclusive de l'autre comme on le verra dans la description ci-après.

Le cas de l'élément de conformation embarqué avec le chariot porte-lame est nécessaire lorsque la lame est inclinée vers l'avant et vers le bas sur son plan de déplacement.

D'autres caractéristiques et avantages de l'invention ressortiront de la description donnée ci-après de plusieurs variantes de sa réalisation.

Il sera fait référence aux dessins annexés parmi lesquels :
- la figure 1 est une vue extérieure d'une forme de réalisation du support général conforme à l'invention,
- la figure 2 est une vue extérieure d'un premier mode de réalisation de cette appareil,
- la figure 3 est une vue en coupe de la figure 2 selon la direction de déplacement de la lame de coupe,
- la figure 4 illustre une variante de réalisation de l'appareil de la figure 3 dans laquelle la lame de coupe et l'élément de conformation de la cornée sont déplaçables à l'unisson,
- la figure 4A est une vue agrandie de détail de la variante de réalisation de la figure 4,
- la figure 4B illustre différents éléments de conformation de la cornée pouvant être associés à la variante de réalisation du dispositif de coupe de la figure 4,
- la figure 5 est une vue en coupe radiale de l'appareil au niveau de la liaison entre la base et le support général,
- la figure 6 est une vue extérieure d'une seconde variante de réalisation de l'invention,
- la figure 7 illustre une autre variante de réalisation de l'appareil selon l'invention.

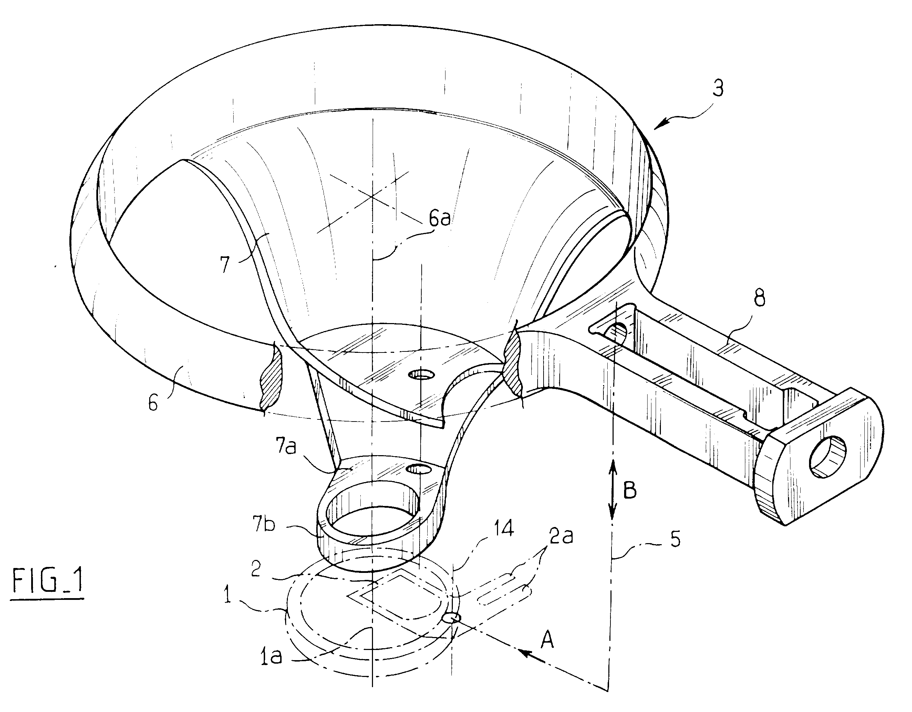

A la figure 1, on a représenté schématiquement par le trait mixte 1 une chambre annulaire à dépression formant la base d'un kératome de chirurgie de la cornée. La chambre annulaire 1 possède un axe de révolution 1a.

De manière connue également un kératome possède une lame de coupe 2 qui est susceptible d'osciller dans un plan perpendiculaire à l'axe 1a de la base et au-dessus de celle-ci. On notera par la suite que cette oscillation s'effectue le long d'un arc de cercle contrairement aux dispositifs connus où elle est rectiligne. Cette lame de coupe 2 lorsqu'elle oscille, est poussée dans le sens A dans son plan d'oscillation en direction de l'axe 1a et c'est dans cette direction que la découpe du disque cornéen s'effectue. Le retrait de la lame s'opère dans la direction inverse à celle représentée à la figure.

Dans l'art antérieur, il existe un porte-lame qui est monté à coulissement dans des rainures supérieures de la chambre à dépression 1 orientées dans le sens A, de manière à guider le mouvement de la lame par rapport à la partie de la cornée qui fait saillie à l'intérieur de la chambre de dépression. En général la lame 2 est précédée d'un plateau qui écrase la cornée juste avant la coupe, plateau porté par le porte-lame. Dans d'autres appareils plus rares, la lame est insérée entre la base et ce plateau de conformation et d'écrasement de la cornée qui est fixe par rapport à la base alors que la lame est mobile entre eux.

Dans l'invention, on a prévu un support général 3 auquel sont reliées d'une part la chambre annulaire à dépression 1 et d'autre part la lame 2 et ce de manière indépendante pour que leurs positons relatives par rapport au support 3 et donc entre elles soient réglables indépendamment l'une de l'autre.

Ainsi la chambre annulaire 1 peut-être réglée en hauteur le long de son axe 1a par rapport au support général 3 grâce à des moyens symbolisés par le trait mixte 4 et qui seront exposés par la suite. La lame 2 est quant à elle reliée au support 3 par des moyens symbolisés par le trait mixte 5 qui permettent d'une part le mouvement selon le sens A de cette lame c'est-à-dire perpendiculairement à l'axe 1a et un réglage dans une direction parallèle à cet axe.

Dans le mode de réalisation représenté, le support général comporte une bague supérieure 6 qui forme un moyen de préhension de l'appareil avec un axe 6a perpendiculaire à son plan.

Un bras 7 s'étend depuis cette bague et sous celle-ci en convergeant vers son axe 6a. L'extrémité libre de ce bras 7 comporte les moyens d'attelage de la base du kératome 1 dans des positions le long de l'axe 6a qui sont telles que l'axe 1a de révolution de cette base coïncide avec l'axe 6a de la bague supérieure 6. Ce bras 7 sera de préférence en forme d'un secteur de paroi conique qui se rétrécit vers sa partie opposée à celle qui le relie à la bague supérieur 6. En outre le support comporte un autre bras 8 qui s'étend à l'extérieur de la bague 6 et qui est perpendiculaire à. son axe 6a, ce bras 8 constituant un organe de support et de guidage en coulissement pour un chariot porte-lame symbolisé par le trait mixte 5, qui peut être ainsi déplacé perpendiculairement à l'axe 1a, le chariot 5 étant extensible dans une direction parallèle à cet axe 1a comme le symbolise la flèche B sur la figure 1.

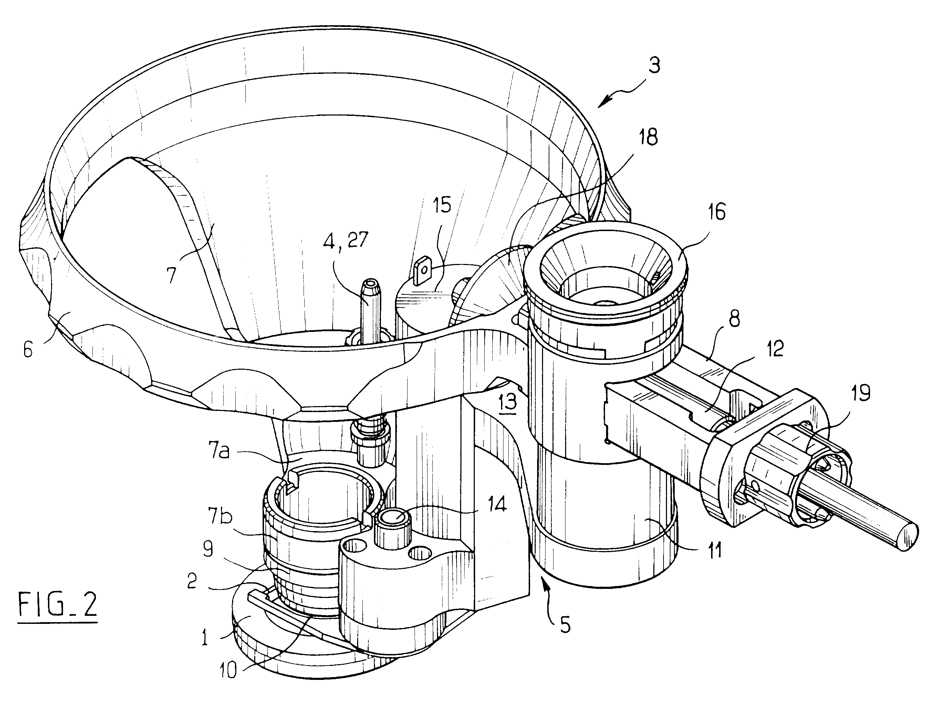

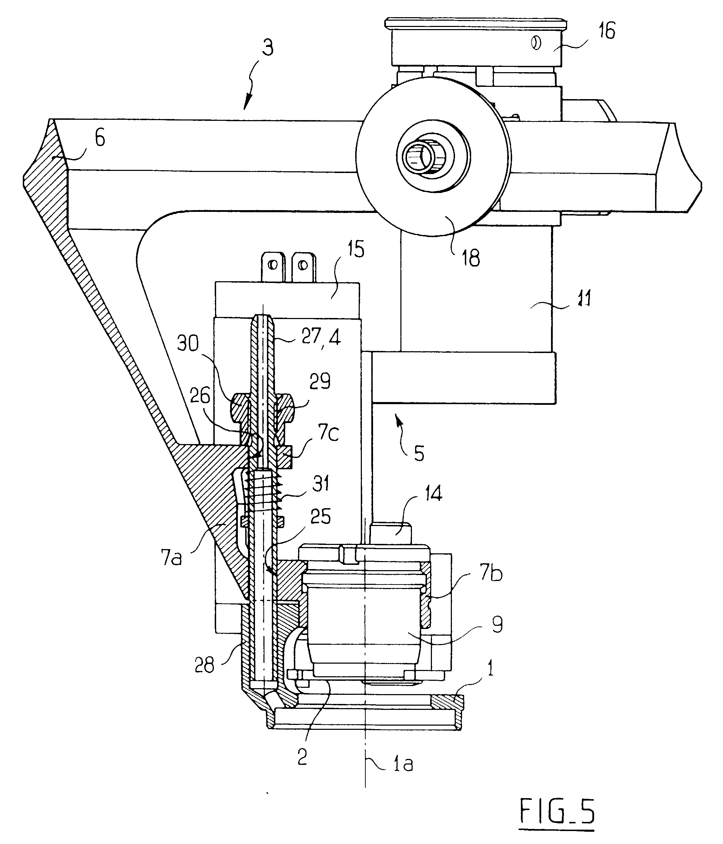

A la figure 2 on a représenté le kératome de l'invention avec l'ensemble de ses constituants. On retrouve sur cette figure les éléments déjà décrits avec les mêmes références. On aura remarqué sur la figure 1 que l'extrémité 7a du bras 7 est conformée en une sorte d'anneau 7b, coaxial à l'anneau 6. Cet anneau 7b est destiné à recevoir une douille 9 fermée en partie inférieure par une paroi transparente de surface 10 qui constitue un élément de conformation de la surface de la cornée, en général un aplatisseur de la cornée, qui est situé en surplomb de la base 1. C'est entre cette surface 10 et la base 1 que le couteau 2 va opérer. La base 1 peut être réglée en hauteur par rapport à cette surface 10 au moyen d'un système vis-écrou 4 dont une description plus détaillé sera faite en regard de la figure 5.

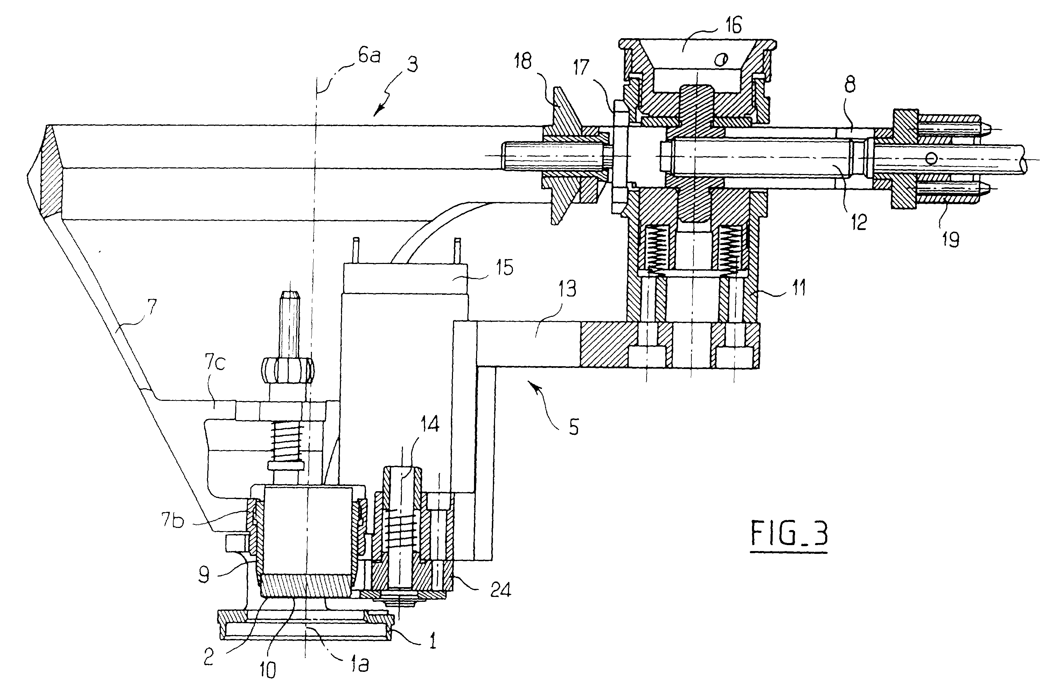

On remarque sur la figure 2 que le chariot porte-lame 5 est de forme assez complexe. Sommairement il comporte un fût vertical 11 dont la partie supérieure est engagée dans le bras 8 et coopère avec une vis 12 à la manière d'un système vis-écrou de sorte qu'une manoeuvre en rotation de cette vis 12, qui est calée longitudinalement dans le guide 8, entraîne un déplacement en translation de ce fût 11 le long du bras 8. Le fût 11 est extensible en longueur comme cela sera d'écrit plus en détail en regard de la figure 3. A la base du fût 11, le chariot possède une partie 13 horizontale qui est orientée en direction de l'anneau 6 sous lequel elle s'étend. Cette partie 13 forment le support pour d'une part un axe 14 de pivotement de la lame 2 et d'autre part un moteur 15 d'entraînement de.cette lame en oscillation autour de cet axe 14. Le moteur 15 est équipé en bout d'arbre de sortie d'un excentrique qui vient s'engager entre les deux branches d'une fourche 2a solidaire de la lame de coupe 2 comme le montre la figure 1 où est également représenté l'axe 14.

La figure 3 est une vue en coupe de la figure 2 par un plan radial qui passe bien entendu par l'axe 6a et par l'axe longitudinal du bras 8 portant le chariot 5 porte-lame. On retrouve sur cette figure certains des éléments déjà décrits avec les mêmes références. On voit sur cette figure par exemple que le fût 11 est suspendu à un écrou 16 qui repose sur le bras 8. On comprend qu'en vissant plus ou moins l'écrou 16 on fasse monter ou descendre le fût 11 par rapport au bras 8, le fût 11 étant bien entendu pourvu d'ouvertures traversées par ce bras 8, avec un jeu permettant de couvrir une plage de réglage suffisante pour l'application requise. On comprend aussi de cette figure qu'en montant ou en descendant le fût 11 par rapport au bras 8, on fait varier la distance séparant la lame 2 de la surface 10 portée par l'extrémité 7a du bras 7 du support général 3. On peut ainsi régler l'épaisseur du disque cornéen que l'on souhaite découper.

On notera également sur cette figure la présence, à l'intérieur du bras 8, d'une butée 17 dont la position à l'intérieur de ce bras est réglable au moyen d'un organe manuel 18. Cette butée constitue une limite de la course du fût 11 et donc du chariot porte-lame 5 en direction de l'axe 6a. Le rôle de cette butée est de permettre de fixer une limite à la découpe du disque cornéen de manière que celui-ci ne soit pas totalement détaché de la cornée, si l'opération l'exige.

La commande de la rotation de la vis 12 qui est la vis de manoeuvre du chariot porte-lame 5 dans son rapprochement ou son éloignement de l'axe 6a, peut-être assurée manuellement par un bouton de commande 19 qui est situé à l'extrémité du bras 8. Cette commande peut être également motorisée en accouplant la vis 12 à un moteur ou un câble d'entraînement connu en lui même.

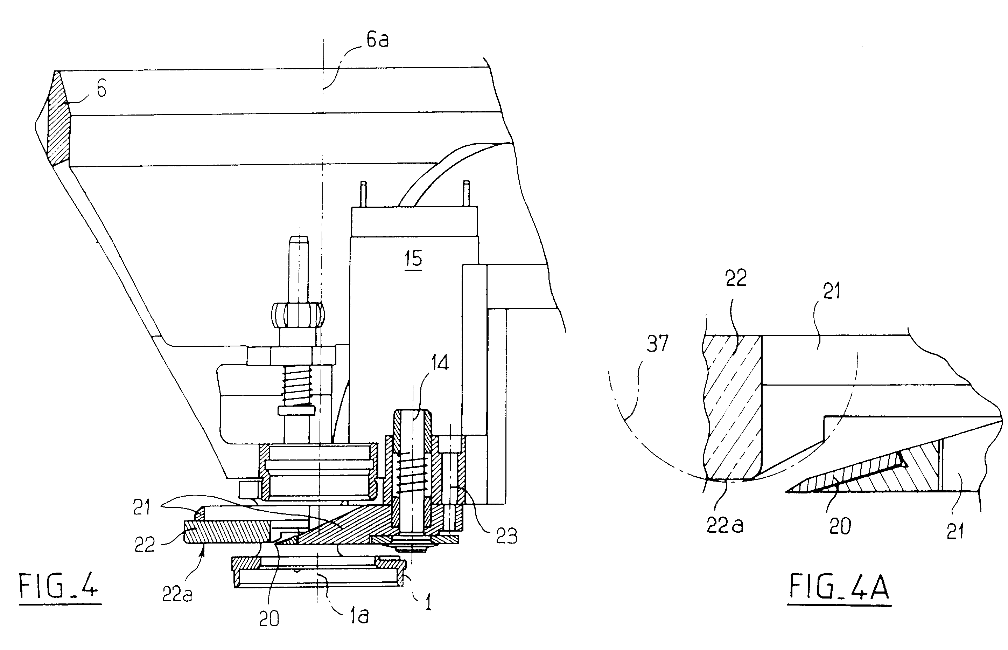

A la figure 4, on a représenté une variante de réalisation du dispositif de coupe. Le couteau possède ici une lame 20 inclinée par rapport à son plan de déplacement perpendiculaire à l'axe 1a, 6a de l'appareil, cette lame étant bien entendu oscillante comme la lame 2 précédemment décrite autour de l'axe 14. Au niveau de cette lame, le chariot possède une extension radiale 21 pour supporter un plateau 22 qui se déplace avec le chariot et dont la surface inférieure 22a constitue la surface d'écrasement de la cornée précédant la lame de découpe comme dans les kératomes connus. On aura noté que, pour passer du kératome selon la figure 3 avec une lame 2 oscillante qui se déplace sous un plateau fixe à une lame 20 oscillante inclinée qui se déplace avec le plateau d'écrasement de la cornée, les opérations d'adaptation sont simples. Il suffit en effet de retirer le douille 9 qui porte la surface 10 de la bague inférieure 7b du support général 3 et de remplacer, grâce à des vis 23, l'élément inférieur 24 du porte-lame de la figure 3 par celui 21 de la figure 4. Dans les conditions de la figure 4, il est possible de régler l'épaisseur du disque cornéen découpé en changeant le plateau 22 donc la distance de sa face inférieure 22a par rapport à la lame de coupe.

La figure 4A est une vue partielle agrandie de la figure 4 au niveau de la lame de coupe 20. On y a représenté en trait mixte 37 un élément de conformation de la cornée qui est différent du plateau d'écrasement 22, ceci pour illustrer le fait qu'on peut mettre en oeuvre soit une surface cylindrique soit une surface en forme de tonneau qui présente au droit du couteau 20 une génératrice non linéaire afin de prendre en compte un certain nombre de défauts particuliers de la cornée comme l'astigmatisme. Bien entendu dans ce cas il conviendra d'orienter l'appareil autour de l'axe 6a en fonction de la topographie de la cornée et notamment de l'orientation du méridien le plus courbe ou le plus plat lorsqu'il y a astigmatisme.

La figure 4B illustre ces différents éléments de conformation de la cornée. L'extension radiale 21 comporte deux bras fendus. Les éléments de conformation sont en forme de rouleaux 38, 39, 40, interchangeables montés à rotation sur des axes internes dont les extrémités sont placées dans les fentes des bras de l'extension 21. Le rouleau 38 qui est monté est en forme de tonneau. Le rouleau 39 est en forme de diabolo. Le rouleau 40 est cylindrique. Les fentes peuvent bien entendu recevoir de manière amovible le plateau 22 au lieu de l'un des rouleaux.

A la figure 5, on a illustré l'attelage de la base 1 au support général 3. Le bras 7 possède à son extrémité 7a non seulement sa portion annulaire 7b pour supporter le manchon 9 mais également une sorte de gousset intermédiaire 7c, l'extrémité 7a et le gousset 7c étant pourvus d'orifices 25,26 alignés, dont l'axe est parallèle à l'axe 1a, et au travers desquels on peut introduire une tige 27 solidaire de la base 1. Par ailleurs, la base 1 est surmontée d'une excroissance 28 qui d'une part sert d'implantation à la tige 27 et d'autre part forme une sorte de semi-collier entourant la partie inférieure de la partie annulaire 7b du bras 7, ce qui constitue un guidage anti-rotation de cette base 1 par rapport à l'axe de la tige 27.

En réalité la tige 27 est creuse et constitue l'embout de raccordement de la chambre à dépression que constitue la base 1 à une source de vide. Une partie supérieure qui s'étend au-delà du gousset 7c de la tige 27 est filetée en 29 de manière à pouvoir coopérer avec un écrou 30 qui s'appuie sur la surface supérieure du gousset. Ainsi la base 1 est-elle suspendue au bras 7. Une manoeuvre de l'écrou 30 permet de régler la hauteur de la base 1 par rapport au bras 7 et un ressort 31 calé entre la surface inférieure 7c et un épaulement de la tige 27 permet de rattraper les jeux et est suffisamment raide pour qu'il n'y ait aucune variation lors de l'opération dans la position relative de la base 1 et de la douille 9.

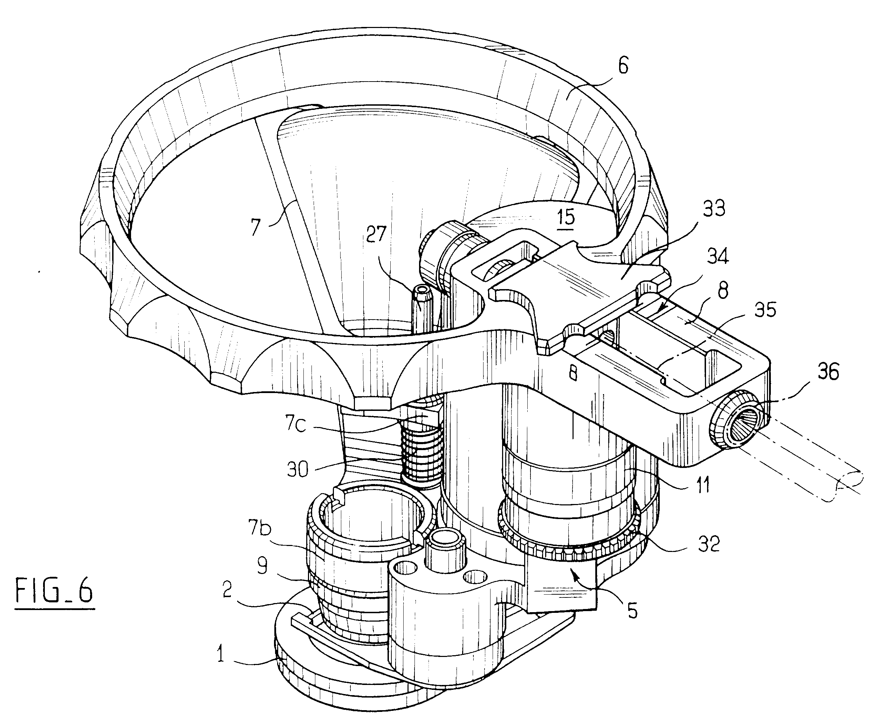

A la figure 6, on a représenté une variante de réalisation du kératome selon l'invention dans laquelle les parties déjà décrites précédemment ont été désignées par les mêmes références. Dans cette variante, le bouton de réglage de la positon de la lame par rapport au support général est situé en dessous du bras 8, et porte la référence 32. Il est ainsi possible de mettre en place sur le bras un curseur 33 qui peut se déplacer le long d'une échelle graduée 34 afin de pouvoir donner une indication au chirurgien sur la course de la lame à comparer avec le diamètre du disque cornéen de manière à conserver une zone de liaison de ce disque à la cornée qui reste un volet plus aisé à rabattre sur le plan de coupe et à remettre en position.

De même, dans cette réalisation, l'entraînement du chariot porte-lame 5 n'est plus réalisé au moyen d'un système vis-écrou mais est motorisé au moyen d'un câble, symbolisé par le trait mixte 35, dont une extrémité est accrochée au chariot porte-lame 5 à l'intérieur du bras 8, ce câble étant mobile longitudinalement à l'intérieur d'une gaine 36 raccordée à l'extrémité du bras 8 et en prolongement de celui-ci. Le câble est manoeuvré en glissement par rapport à la gaine au moyen d'un moteur connu qui agit à l'autre extrémité de cette gaine. Il s'agit là d'un mécanisme d'entraînement connu en lui-même qui présente l'avantage de commander de manière très régulière le déplacement du chariot 5 le long du bras 8.

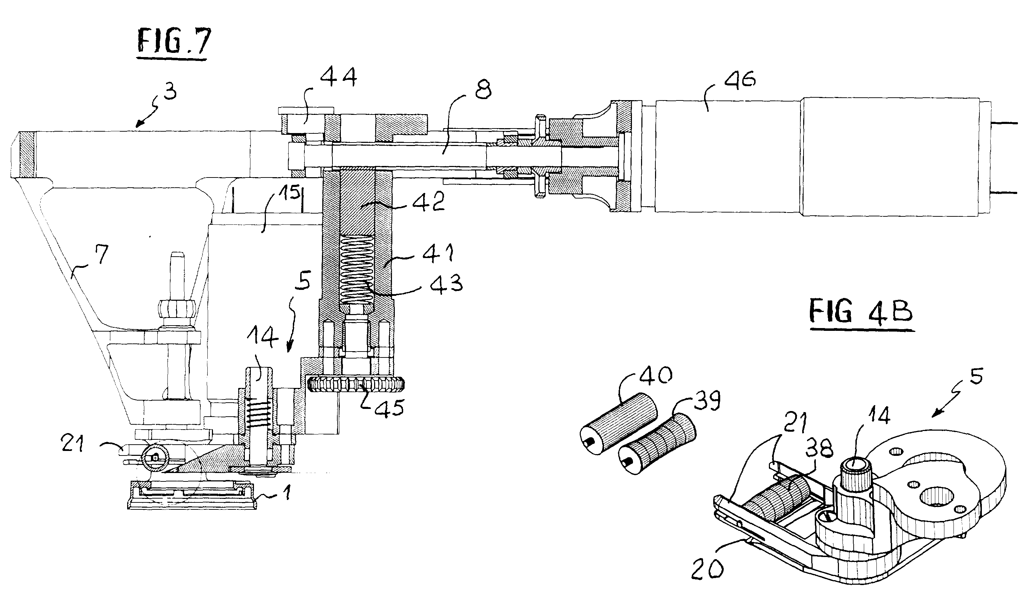

A la figure 7, on retrouve la plupart des éléments déjà décrits avec les mêmes références. La variante représentée est destinée à la découpe de la cornée par une lame inclinée. Il n'est alors plus nécessaire de disposer d'un réglage en hauteur comme celui du fût 11. Le fût 41 est dans ce cas directement en appui sur le bras 8. A l'intérieur du fût 41 il existe un demi-écrou 42 qui est poussé en permanence contre la vis 12 par un ressort 43. Quand le fût 41 arrive en butée contre l'élément d'arrêt réglable 44, si la vis 12 continue de tourner, le demi-écrou 42 échappe au filetage de la vis ce qui permet de débrayer la liaison vis-écrou.

Un bouton 45 assure la fixation du chariot 5 au fût 41. L'élément d'arrêt réglable 44 diffère de la butée 17 de la figure 3 en ce qu'il est constitué par une surface de came, par exemple un excentrique.

On notera enfin la présence d'un moteur 46 directement accouplé à la vis 12, à l'extrémité du bras 8 par tout moyen connu déconnectable.

## Revendications

1. Appareil chirurgical pour réaliser une découpe lamellaire de la cornée, comportant :
- une base (1) qui comprend une chambre à dépression annulaire avec un axe de révolution (1a) conformée pour être appliquée sur la sclère d'un oeil et pourvue de moyens (27) pour sa connexion à une source de vide,
- un élément de conformation de la cornée situé au dessus de la base (1) et présentant une surface (10 ; 22a) tournée en regard de celle-ci,
- une lame de coupe (2 ; 20) déplaçable dans un plan situé entre la surface (10 ; 22a) de l'élément de conformation et la base (1),
caractérisé en ce qu'il comporte un support général (3) auquel est attelée la base (1) par des moyens (4 ; 27) lui permettant d'être réglée en position par rapport à ce support (3) le long de l'axe de révolution (1a) de la chambre annulaire et auquel est attelée la lame (2) de coupe par des moyens (5) lui permettant d'être réglée en position par rapport au support général (3) le long d'une direction parallèle à l'axe (1a) de révolution de la chambre annulaire et d'être déplacée perpendiculairement à cet axe (1a).

2. Appareil selon la revendication 1 caractérisé en ce que le support général (3) comporte une bague supérieure (6) formant un moyen de préhension de l'appareil, avec un axe (6a) perpendiculaire à son plan, un bras (7) s'étendant à partir de la bague (6) sous celle ci en convergeant vers son axe (6a), par l'extrémité libre (7a) duquel la base (1) est maintenue dans des positions réglables telles que son axe de révolution (1a) coïncide avec l'axe (6a) de la bague supérieure (6) et un bras (8) qui s'étend à partir de la bague (6) à l'extérieur de celle-ci et perpendiculairement à son axe, sur lequel est monté à coulissement un chariot (5) porte-lame extensible (B) de manière réglable dans une direction parallèle à l'axe (6a) de la bague (6).

3. Appareil selon la revendication 2 caractérisé en ce que l'élément de conformation de la cornée est constitué par une lentille (9,10) rapportée de manière fixe mais démontable au bras convergent (7) du support général (3).

4. Appareil selon la revendication 2 caractérisé en ce que l'élément de conformation (22,37) est solidaire du chariot porte-lame (5), en avant de la lame (2) dans le mouvement qui tend à rapprocher cette lame (2) de l'axe (6a) du support général (3).

5. Appareil selon la revendication 4 caractérisé en ce que la lame (20) est inclinée vers l'avant et vers le bas sur son plan de déplacement.

6. Appareil selon l'une des revendications précédentes caractérisé en ce que le support comporte une butée réglable (17) de limitation du mouvement du chariot (5) porte-lame en direction de l'axe (6a).

7. Appareil selon l'une des revendications précédente caractérisé en ce que la lame (2, 20) de coupe est montée oscillante dans son plan de déplacement par rapport au chariot porte-lame (5), le chariot portant un organe moteur (15) d'actionnement de la lame (2) par l' intermédiaire d'une came excentrique.

8. Appareil selon l'une des revendications précédente caractérisé en ce que les moyens (27) de connexion de la chambre à dépression annulaire de la base (1) comportent un embout cylindrique formant vis (29) d'un système vis-écrou (29,30) qui constitue l'organe de réglage de la position de la base (1) par rapport au support général (3).

9. Appareil selon la revendication 4 caractérisé en ce que l'élément de conformation (38, 39, 40) de la cornée possède en regard de la base une surface cylindrique ou en forme de tonneau.

10. Appareil selon la revendication 2 caractérisé en ce que le chariot (5) porte-lame est attelé à un organe d'entraînement le long du bras (8) formé par un câble (35) mobile en translation dans une gaine (36) attelé au bras (8) et en prolongement de celui-ci.

11. Appareil selon la revendication 2, caractérisé en ce que le chariot (5) porte-lame est attelé à un organe d'entraînement le long du bras (8) formé par une vis (12) mobile en rotation dans le bras et attelé à un moteur (46) de son entraînement dans le prolongement du bras (8).

12. Appareil selon la revendication 11, caractérisé en ce que la vis (12) est en prise avec un demi-écrou débrayable (42) solidaire du chariot porte-lame.
